# EUROPÄISCHE PATENTSCHRIFT

(11) **EP 3 119 279 B1**
(45) Veröffentlichungstag und Bekanntmachung des Hinweises auf die Patenterteilung: **07.03.2018**
(21) Anmeldenummer: 14710890.6
(22) Anmeldetag: 18.03.2014
(51) Int. Cl.: A61B 5/11, E04F 15/024, A61B 5/00, E04F 15/02

(54) **BODENAUFBAU**
FLOOR STRUCTURE
STRUCTURE DE PLANCHER

(43) Veröffentlichungstag der Anmeldung: 25.01.2017
(73) Patentinhaber: RK Rose + Krieger GmbH Verbindungs- und Positioniersysteme, 32423 Minden (DE)
(72) Erfinder: BLAB, Manfred, 88048 Friedrichshafen-Ailingen (DE)
(74) Vertreter: Dantz, Jan Henning
(86) Internationale Anmeldenummer: PCT/EP2014/055395
(87) Internationale Veröffentlichungsnummer: WO 2015/139734

(56) Entgegenhaltungen:
- EP-A2- 1 760 222
- CN-A- 1 561 908
- US-A- 4 600 016
- US-A- 5 787 663

## Beschreibung

Die vorliegende Erfindung betrifft einen Bodenaufbau, insbesondere für ein Ganglabor, mit Längsstreben und Querstreben.

US 5 787 663, das als nächstliegender Stand der Technik angesehen wird, offenbart einen Bodenaufbau nach dem Oberbegriff des Anspruchs 1.

Ein weiteres Dokument, EP 1 760 222, offenbart einen demontierbaren Bodenaufbau, bei dem an Füßen horizontale Profile eingehängt werden. Auf die Profile werden dann Anschlagselemente montiert, um Bodenplatten aufzulegen. Ein solcher Bodenaufbau eignet sich für den Privatbereich, besitzt allerdings keine ausreichende Stabilität, um in einem Labor eingesetzt zu werden. Denn die lose eingehängten horizontalen Profile besitzen ein gewisses Spiel, was dem Bodenaufbau nicht die ausreichende Stabilität für Druckmessungen gibt.
Für die Messung in Ganglaboren ist es bekannt, Kraftmessplatten mittels eines Montagerahmens in eine Vertiefung eines Bodens einzusetzen. Untersuchungen zum Einfluss von Deckenschwingungen auf die Messgenauigkeit in Ganglaboren (Beitrag zur Tagung für Erdbebeningenieurwesen und Baudynamik 2013) haben ergeben, dass die personeninduzierten Schwingungen in die Bauwerksdecke sich nicht signifikant auf die Messgenauigkeit auswirken. Allerdings ist das Einlassen einer einzelnen Kraftmessplatte mit dem Montagerahmen in eine Vertiefung eines Bodens vergleichsweise aufwändig. Zudem besteht das Problem, dass die Messplatte optisch sichtbar ist, was das Verhalten der Probanden beeinflussen kann.
Ferner sind Laufbänder für Analysen bekannt. Solche Laufbänder sind jedoch schlecht geeignet, da keine genügend große Strecke für eine ausreichende Analyse vorhanden ist und die Ganggeschwindigkeit nicht selbst gewählt werden kann, so dass das Laufverhalten sich von dem Laufverhalten auf der freien Fläche unterscheidet.
Es ist daher Aufgabe der vorliegenden Erfindung, einen Bodenaufbau zu schaffen, der zur Durchführung von Druckmessungen geeignet ist und zudem einfach montiert werden kann.

Diese Aufgabe wird mit einem Bodenaufbau mit den Merkmalen des Anspruches 1 gelöst.

Bei dem erfindungsgemäßen Bodenaufbau sind die Längsstreben und Querstreben über lösbare Klemmverbinder aneinander festgelegt. Dadurch bilden Längsstreben und Querstreben eine stabile Einheit, da sie kraftschlüssig aneinander fixiert sind. Ferner ist eine Vielzahl von Haltern an den Längsstreben und Querstreben fixiert, auf denen Bodenplatten abgelegt sind, wobei zumindest eine der Bodenplatten als Messplatte zur Kraftmessung ausgebildet ist. Der erfindungsgemäße Bodenaufbau lässt sich somit einfach an oder in einem Bauwerk montieren und demontieren, je nachdem, ob entsprechende Messungen nur temporär oder dauerhaft durchgeführt werden sollen. Durch die Verwendung von Bodenplatten für den gesamten Bodenaufbau kann die Oberfläche so gestaltet werden, dass optisch nicht erkennbar ist, welche der Bodenplatten als Messplatte und welche der Bodenplatten nur als Bodenplatten ohne Messfunktion ausgebildet sind. Dies erleichtert den natürlichen Bewegungsablauf von Probanden.

Für eine stabile Befestigung der Längsstreben und der Querstreben können diese über Stoßverbinder und/oder Eckverbinder aneinander festgelegt sein. Die Stoßverbinder und/oder Eckverbinder können mit den Längsstreben und den Querstreben verschraubt oder anderweitig klemmend fixiert sein. Es ist auch möglich, die Längsstreben und die Querstreben nur über die Halter aneinander zu fixieren.

Auch die Halter können über Klemmelemente an den Längsstreben und/oder Querstreben festgelegt sein. Beispielsweise können die Klemmelemente als Nutensteine ausgebildet sein, die an den Längsstreben und/oder Querstreben fixiert werden.

Um den Einfluss auf die Messplatte durch Eigenschwingungen des Bodenaufbaus zu minimieren, kann zwischen den Haltern und den Bodenplatten ohne Messfunktion zumindest bereichsweise mindestens ein Dämpfungselement angeordnet sein. Vorzugsweise ist an jedem Halter ein Dämpfungselement, beispielsweise durch eine elastische Dämpfungsauflage, vorgesehen, das verhindert, dass Schwingungen in die Längsstreben und die Querstreben eingebracht werden. Zudem besitzen die Bodenplatten zur Vermeidung von Schwingungen ein hohes Eigengewicht, beispielsweise von mehr als 15 kg, insbesondere mehr als 20 kg, so dass der Bodenaufbau im Wesentlichen starr ist.

Die Längsstreben und/oder Querstreben bestehen vorzugsweise aus Metall, insbesondere aus Aluminium. Ferner können die Längsstreben und/oder Querstreben hinterschnittene Nuten aufweisen, an denen Nutensteine klemmend festgelegt sind. Dabei kann an zwei Seiten, vorzugsweise an allen vier Seiten bei einem im Querschnitt rechteckförmigen oder quadratischen Profil jeweils eine Nut vorgesehen sein. Solche Profile lassen sich effektiv durch Extrusion herstellen und ermöglichen eine stabile Verbindung.

Die erfindungsgemäße Messplatte weist vorzugsweise eine Vielzahl von Drucksensoren auf. Die Drucksensoren können durch piezometrische Messaufnehmer gebildet sein.

Die Halter bestehen vorzugsweise aus Metall, insbesondere aus Stahl und Aluminium, und weisen eine oder mehrere Gewindebohrungen auf, um zumindest die Messplatten daran anzuschrauben. Die Bodenplatten ohne Messfunktion können auch lose auf die Halter aufgesteckt werden, da geringfügige Bewegungen der Bodenplatten ohne Messfunktion die Messergebnisse nicht nachteilig beeinflussen.

Ferner ist es möglich, die Längsstreben und/oder die Querstreben mit dem darunter angeordneten Boden zu verbinden, insbesondere zu verschrauben, wobei dann entsprechende Halter an den Längsstreben und/oder Querstreben kraftschlüssig fixiert sind.

Der Bodenaufbau kann eine einzelne Messplatte zur Kraftmessung oder mehrere Messplatten zur Kraftmessung aufweisen. Vorzugsweise ist die Oberfläche der Bodenplatten mit Messfunktion identisch ausgebildet zur Oberfläche der Bodenplatten ohne Messfunktion, so dass für Probanden nicht erkennbar ist, an welchen Bodenplatten eine Messung vorgenommen wird.

Der erfindungsgemäße Bodenaufbau wird vorzugsweise für ein Ganglabor in Verbindung mit optischen Erfassungsmitteln verwendet. In einem Ganglabor werden medizinische Gang- und Laufanalysen durchgeführt, um Bewegungsabläufe bei Patienten oder Sportlern zu analysieren. Über die Messplatten kann eine präzise Messung der vom Menschen auf die Bodenplatte übertragenen Kräfte erhalten werden. Der Bodenaufbau kann dabei auch mobil ausgestaltet sein, so dass dieser beispielsweise in Montagehallen und Produktionshallen eingesetzt werden kann, um die Abläufe zum Beispiel bei einer Montage ergonomisch zu vermessen, um dann eine Optimierung vornehmen zu können. Der Bodenaufbau kann dabei selbsttragend stabil ausgeführt sein und mobil eingesetzt werden, da eine Zerlegung in Einzelteile für den Transport und der Aufbau an einem anderen Ort problemlos möglich sind.

Die Erfindung wird nachfolgend anhand eines Ausführungsbeispiels mit Bezug auf die beigefügten Zeichnungen näher erläutert. Es zeigen:
- Figur 1: eine Draufsicht auf einen erfindungsgemäßen Bodenaufbau mit teilweise abgenommenen Bodenplatten;
- Figur 2: eine perspektivische Ansicht des Bodenaufbaus der Figur 1;
- Figur 3: eine Seitenansicht des Bodenaufbaus der Figur 1;
- Figur 4: eine vergrößerte Detailansicht des Bodenaufbaus der Figur 1 im Bereich eines Halters;
- Figuren 5A und 5B: zwei perspektivische Ansichten eines Halters des Bodenaufbaus der Figur 1;
- Figur 6: eine perspektivische Ansicht einer Messplatte des Bodenaufbaus der Figur 1;
- Figur 7: eine Schnittansicht durch den Bodenaufbau der Figur 1 im Bereich eines Halters;
- Figur 8: eine Draufsicht auf einen Bodenaufbau gemäß einer modifizierten Ausführungsform;
- Figur 9: eine Seitenansicht auf den Bodenaufbau der Figur 8, und
- Figur 10: eine Vorderansicht auf den Bodenaufbau der Figur 8.

Ein Bodenaufbau 1 umfasst eine Vielzahl von parallel ausgerichteten Längsstreben 2 und senkrecht dazu verlaufenden Querstreben 3. Die Längsstreben 2 und Querstreben 3 sind als extrudierte Aluminiumprofile ausgebildet und besitzen an allen vier Seiten hinterschnittene Nuten zur Befestigung von Nutensteinen oder anderen Befestigungsmitteln. Die Längsstreben 2 und Querstreben 3 sind an ihrem Verbindungsbereich über Stoßverbinder oder Eckverbinder miteinander kraftschlüssig verbunden.

An der Oberseite der Längsstreben 2 und der Querstreben 3 sind mehrere Halter 4 vorgesehen, die ebenfalls aus Metall, insbesondere aus Stahl oder Aluminium, hergestellt sind, und eine Auflagefläche für Bodenplatten 5 ausbilden. Zwischen den Haltern 4 und den Bodenplatten 5 können noch Dämpfungselemente aus elastischem Material angeordnet sein, um beim Belasten der Bodenplatten 5 das Einbringen von Schwingungen in die Längsstreben 2 und die Querstreben 3 zu reduzieren.

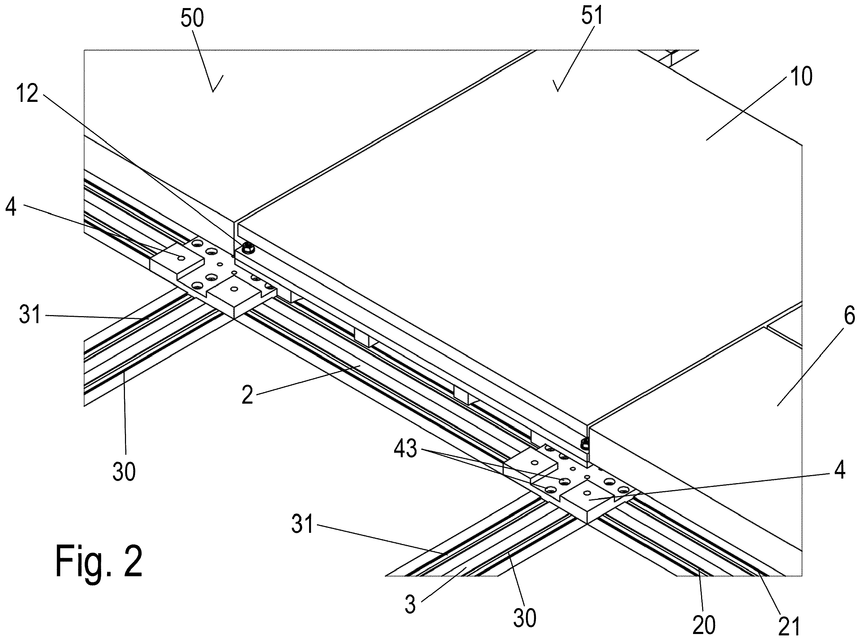

Bei dem Bodenaufbau 1 ist zumindest eine Bodenplatte als Messplatte 10 ausgebildet, wobei eine Oberfläche 51 der Messplatte 10 identisch zu einer Oberfläche 50 einer Bodenplatte 5 ohne Messfunktion ausgebildet ist, um ein einheitliches Oberflächenbild zu erzeugen und einem Proband nicht zu signalisieren, an welcher Stelle eine Messung vorgenommen wird, damit die Bewegungsabläufe nicht beeinflusst werden. Wie in Figur 2 gezeigt ist, wird die Messplatte 10 über Schrauben 12 an dem Halter 4 kraftschlüssig festgelegt, wobei die Messplatte 10 einen Rahmen 11 aus Metall, insbesondere aus Stahl, aufweist, der über Schrauben 12 an vier Ecken kraftschlüssig festgelegt wird.

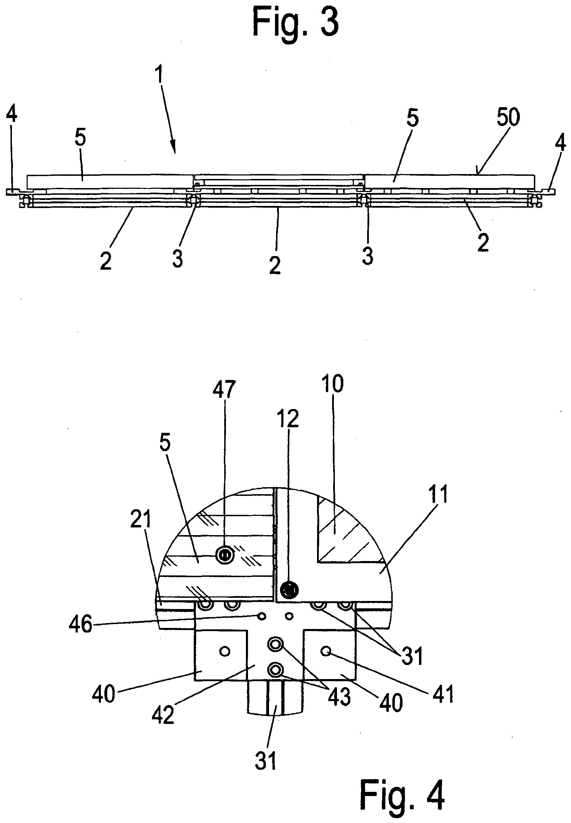

In Figur 3 ist eine Seitenansicht des Bodenaufbaus 1 gezeigt. Die Längsstreben 2 und die Querstreben 3 können im Querschnitt identisch ausgebildet sein und sind an ihren Verbindungsstellen kraftschlüssig aneinander fixiert, insbesondere über Nutensteine, die an Stoßverbindern oder Eckverbindern festgelegt werden. Dadurch wird ein formstabiles Gerüst aus Längsstreben 2 und Querstreben 3 hergestellt, an denen die Halter 4 an der Oberseite ebenfalls kraftschlüssig fixiert sind. Es ist auch möglich, die Längsstreben 2 und die Querstreben 3 nur über die Halter 4 aneinander kraftschlüssig zu fixieren.

In Figur 4 ist der Bereich eines Halters 4 im Detail dargestellt. Jeder Halter 4 umfasst vier nach oben hervorstehende Platten 40, die über eine kreuzförmige Ausnehmung 42 voneinander getrennt sind. An jeder Platte 40 ist eine Gewindebohrung 41 ausgebildet. In die Gewindebohrung 41 kann ein Gewindebolzen 47 eingeschraubt sein, auf den eine Bodenplatte 5 lose aufgesteckt wird. Die Bodenplatte 5 kann einen Kern aus einer MDF- oder HDF-Platte oder einem anderen Material aufweisen und besitzt eine Dicke, die eine Durchbiegung verhindert. Die Bodenplatte 5 ist dabei an vier Eckbereichen auf einer Platte 40 eines Halters 4 abgestützt, wobei die Bodenplatte 5 auf die Gewindebolzen 44 lose aufgesteckt werden kann. Aufgrund des Eigengewichtes der Bodenplatten 5 und der Auflage auf ein elastisches Dämpfungsmaterial ist ein kraftschlüssiges Fixieren nicht notwendig. Dennoch können Befestigungsmittel zum kraftschlüssigen Fixieren auch der Bodenplatten 5 vorgesehen sein.

An dem Halter 4 ist ferner eine kreuzförmige Ausnehmung 42 vorgesehen, an der weitere Öffnungen 43 vorgesehen sind. In den Öffnungen 43 können Befestigungselemente für Nutensteine fixiert werden, um den Halter 4 kraftschlüssig an den Längsstreben 2 und/oder den Querstreben 3 zu fixieren. Im Bereich der kreuzförmigen Ausnehmung 42 sind in einem mittleren Bereich ferner Gewindebohrungen 46 vorgesehen, die zum Eindrehen von Schrauben 12 der Messplatte 10 dienen. In Figur 4 ist von der Messplatte 10 der metallische Rahmen 11 erkennbar, der über die Schraube 12 an dem Halter 4 fixiert ist.

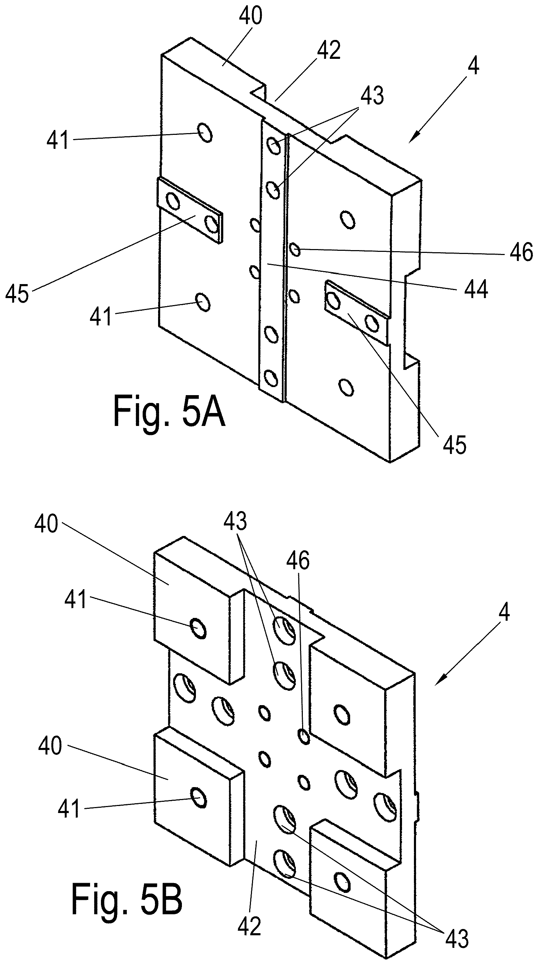

In den Figuren 5A und 5B ist ein Halter 4 im Detail gezeigt. An der Oberseite des Halters 4 sind vier Platten 40 vorgesehen, die über eine kreuzförmige Ausnehmung 42 voneinander beabstandet sind. Ferner sind in den Platten 40 die Gewindebohrungen 41 und in der kreuzförmigen Ausnehmung 42 die Öffnungen 43 zu erkennen, die ebenfalls als Gewindebohrungen ausgebildet sein können. Auf der Unterseite des Halters 4 ist eine nach unten hervorstehende Rippe 44 vorgesehen, die von den Öffnungen 43 durchgriffen ist. Die Rippe 44 kann in eine obere Nut 21 oder eine obere Nut 31 einer Längsstrebe 2 oder einer Querstrebe 3 eingefügt werden. Senkrecht zu der Rippe 44 sind zwei beabstandet angeordnete Rippen 45 an der Unterseite vorgesehen, die ebenfalls in eine Nut 21 oder 31 an der Oberseite einer Längsstrebe 2 oder Querstrebe 3 eingefügt werden können. Die Rippen 44 und 45 erleichtern die Zentrierung der Halter 4.

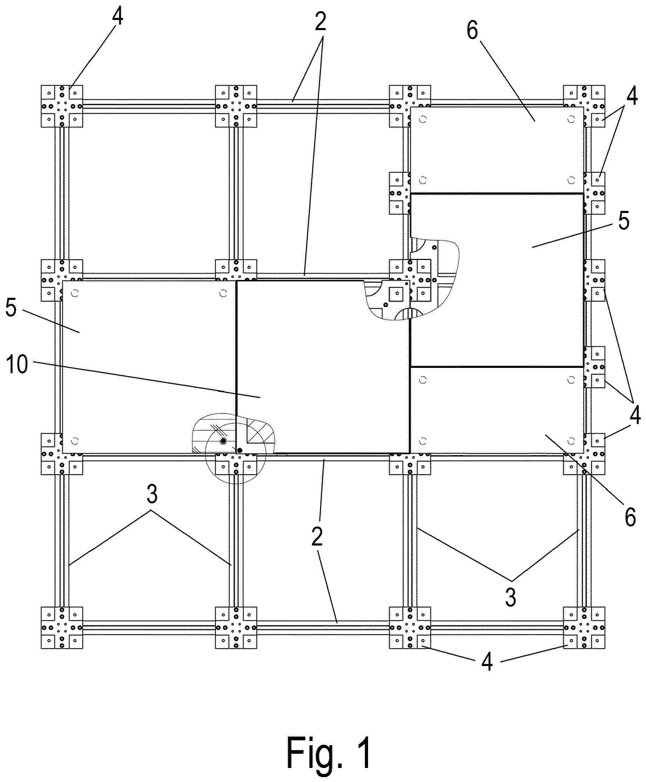

Die Halter 4 müssen nicht unbedingt im Kreuzungsbereich einer Längsstrebe 2 und einer Querstrebe 3 montiert sein. Es ist auch möglich, die Halter 4 in Zwischenabständen zu montieren, wie dies in Figur 1 in einem rechten Abschnitt des Bodenaufbaus 1 dargestellt ist. Die Bodenplatte 5 ist von zwei kleineren Bodenplatten 6 umgeben, die etwa die halbe Größe wie die Bodenplatte 5 besitzen. Daher sind die Halter 4 an den Querstreben 3 in diesem Abschnitt in unregelmäßigen Abständen befestigt.

Bei dem Bodenaufbau 1 können die Halter 4 völlig flexibel entlang der Längsstreben 2 und der Querstreben 3 verschoben und dann fixiert werden. Dadurch können auch andere Geometrien mit dem Bodenaufbau 1 abgedeckt werden als eine Rechteckform.

Zudem ist es auch möglich, den Bodenaufbau 1 nicht eben auszubilden, sondern stufenförmig. Hierfür können entsprechende Stufen an den Längsstreben 2 und den Querstreben 3 kraftschlüssig montiert werden. Dann kann eine Vermessung eines Bewegungsablaufes nicht nur in der Ebene, sondern auch beim Begehen von Stufen erfolgen. Gleichermaßen können auch schräge Ebenen oder andere Geometrien mit den Bodenplatten 5 und 10 abgedeckt werden.

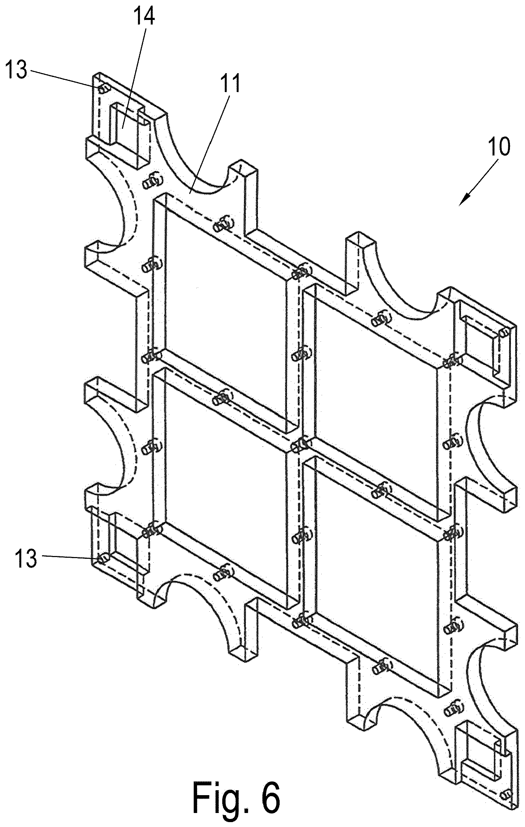

In Figur 6 ist der Rahmen 11 einer Messplatte 10 gezeigt. Der Rahmen 11 aus Metall weist an vier Ecken jeweils Ausnehmungen 14 auf, in die die Platten 40 eingefügt werden können. Ferner sind im Eckbereich jeweils Öffnungen 13 für Befestigungsmittel, insbesondere Schrauben, vorgesehen, um diese an einer Gewindebohrung 46 eines Halters 4 zu verschrauben. An dem Rahmen 11 werden eine Vielzahl von Drucksensoren, insbesondere piezoelektrische Elemente, eingesetzt, die eine mechanische Belastung über eine Druckmessung erfassen, wobei Kräfte in unterschiedliche Richtungen gemessen werden, sowohl in vertikale als auch in horizontale Richtung. Daher liegen die Bodenplatte 5 und die Messplatte 10 mit einem geringfügigen Spalt benachbart zueinander, um an der Messplatte 10 entsprechend genaue Messungen vornehmen zu können.

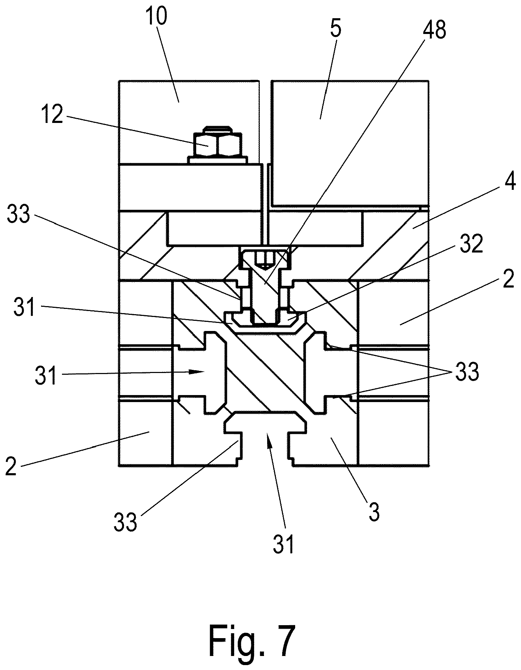

In Figur 7 ist ein Schnitt durch den Bodenaufbau der Figur 1 im Bereich eines Halters 4 gezeigt. Der Halter 4 ist über eine Schraube 48 an einer Längsstrebe 2 fixiert, wobei hierfür ein Nutenstein 32 in die obere Nut 31 der Längsstrebe 2 eingefügt ist. Die Längsstrebe 2 ist im Wesentlichen im Querschnitt quadratisch und umfasst an jeder Seite eine Nut 31, die hinterschnitten ausgebildet ist, wobei im äußeren Bereich nach innen ragende Stege 33 vorgesehen sind, so dass Nutensteine 32 die Stege 33 hintergreifen. Die Nutensteine 32 können in Längsrichtung der Längsstrebe 2 beliebig verschoben und über Schrauben 48 fixiert werden, so dass ein sehr flexibler Bodenaufbau möglich ist.

Der erfindungsgemäße Bodenaufbau wird insbesondere in einem Ganglabor zur Durchführung von Gang- und Laufanalysen eingesetzt. Über eine oder mehrere Messplatten 10 kann eine präzise Messung der vom Menschen auf den Boden übertragenen Kräfte vorgenommen werden, wobei die Kräftemessung insbesondere durch Erfassung von Bewegungsabläufen über optische Erfassungsmittel, insbesondere Kameras, ergänzt wird. Solche Ganglabore können auch für den Sportbereich oder orthopädische Zwecke eingesetzt werden.

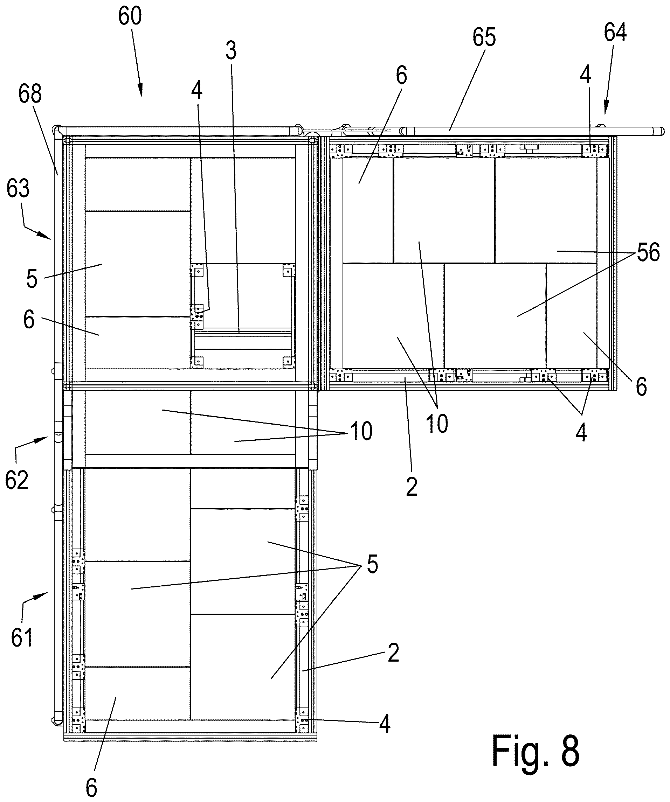

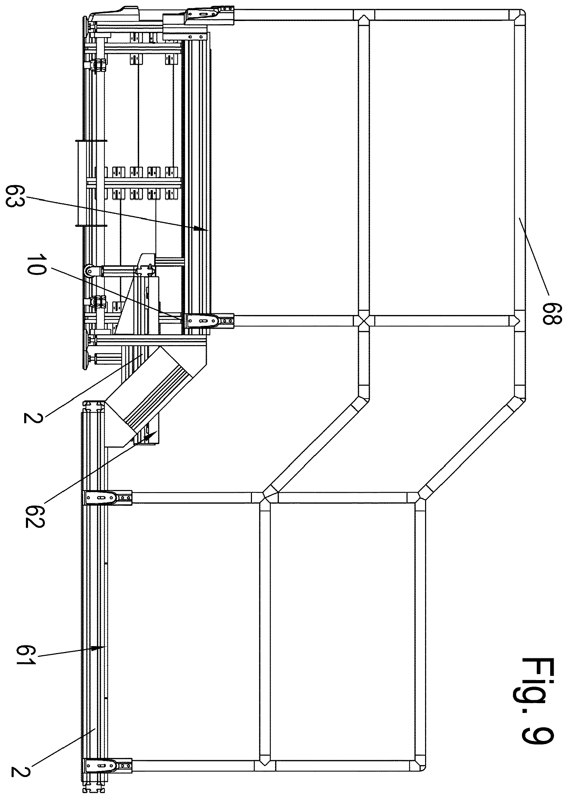

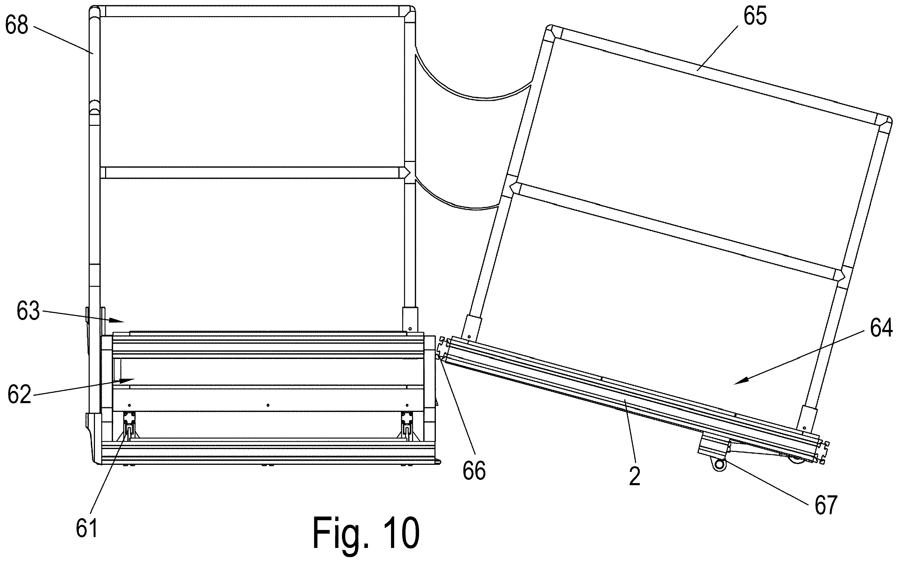

In den Figuren 8 bis 10 ist ein modifizierter Bodenaufbau 60 gezeigt, bei dem eine Bodenfläche nicht in einer Ebene angeordnet ist, sondern stufenförmig ansteigend oder abfallend. Hierfür umfasst der Bodenaufbau 60 einen unteren Sockel 61, beidem eine Unterkonstruktion aus Längsstreben 2 und Querstreben 3 vorgesehen ist, auf denen Bodenplatten 5 fixiert sind, die teilweise auch als Messplatten 10 ausgebildet sein können. Die Festlegung der Bodenplatten 5 und der Messplatte 10 erfolgt wie bei dem vorangegangenen Ausführungsbeispiel über Halter 4. Neben dem Sockel 61 ist eine gegenüber dem Sockel 61 erhöht angeordnete Stufe 62 ausgebildet, bei der zwei Messplatten 10 fixiert sind. Der Bodenaufbau 60 umfasst ferner ein Podest 63, das oberhalb der Stufe 62 angeordnet ist. An dem Podest 63 sind Bodenplatten 5 an Längsstreben 2 und Querstreben 3 über Halter 4 fixiert, wie dies mit Bezug auf Figur 1 schon erläutert wurde. Ferner können statt der quadratischen Bodenplatten 5 auch schmalere rechteckförmige Bodenplatten 6 an der Unterkonstruktion aus Längsstreben 2 und Querstreben 3 fixiert werden.

Von dem Sockel 63 erstreckt sich eine Rampe 64 schräg nach unten, die über einen Halter 66 mit einer Längsstrebe 2 im Bereich des Podestes 63 verbunden ist. Auf der vom Podest 63 abgewandten Seite ist ein Fußelement 67 an einer Unterseite einer Längsstrebe 2 fixiert, wobei das Fußelement 67 Rollen oder andere Mittel aufweisen kann, um die Rampe 64 bei Bedarf verfahren zu können. Um ein Stürzen von Probanden zu vermeiden, ist an der Rampe 64 ein Geländer 65 fixiert, das über entsprechende Verbindungsmittel ebenfalls an den Längsstreben 2 und/oder den Querstreben 3 fixiert sein kann. Im Bereich des Podestes 63 ist ebenfalls ein winkelförmiges Geländer 68 vorgesehen und im Bereich der Stufe 62 und dem Sockel 61 ist ebenfalls ein Geländer angeordnet.

Über den Bodenaufbau 60 gemäß Figur 8 können Untersuchungen zum Gangverhalten auch im Bereich von Stufen 62 oder einer Rampe 64 durchgeführt werden. Insbesondere für orthopädische Analysen können solche Aufbauten erforderlich sein, um für Patienten eine optimale Behandlung vornehmen zu können, damit diese nicht nur in einer Ebene, sondern auch entlang einer Rampe 64 und an Stufen 62 im Bewegungsverhalten analysiert werden können. Aufgrund des flexiblen Aufbaus mit Längsstreben 2 und Querstreben 3, die beliebig über Nutensteine und andere Befestigungsmittel aneinander fixiert werden können, lassen sich flexible Bodenaufbauten realisieren, die für entsprechende Analysen eingesetzt werden.

Insbesondere können auch Bodenaufbauten hergestellt werden, die so zusammengesetzt werden, dass sie einen Montageablauf einer Produktion simulieren können. Dann kann der Bodenaufbau auch zur Analyse von Montageabläufen und der Bewegungen der Monteure eingesetzt werden.

### Bezugszeichenliste

- 1: Bodenaufbau
- 2: Längsstrebe
- 3: Querstrebe
- 4: Halter
- 5: Bodenplatte
- 6: Bodenplatte
- 10: Messplatte
- 11: Rahmen
- 12: Schraube
- 13: Öffnung
- 14: Ausnehmung
- 21: Nut
- 31: Nut
- 32: Nutenstein
- 33: Steg
- 40: Platte
- 41: Gewindebohrung
- 42: Ausnehmung
- 43: Öffnung
- 44: Rippe
- 45: Rippe
- 46: Gewindebohrung
- 47: Gewindebolzen
- 48: Schraube
- 50: Oberfläche
- 51: Oberfläche
- 60: Bodenaufbau
- 61: Sockel
- 62: Stufe
- 63: Podest
- 64: Rampe
- 65: Geländer
- 66: Halter
- 67: Fußelement
- 68: Geländer

## Patentansprüche

1. Bodenaufbau (1), insbesondere für ein Ganglabor, mit Längsstreben (2) und Querstreben (3), die über lösbare Klemmverbinder aneinander festgelegt sind, und an denen eine Vielzahl von Haltern (4) fixiert sind, auf denen Bodenplatten (5, 10) abgelegt sind,
**dadurch gekennzeichnet, dass**
zumindest eine der Bodenplatten als Messplatte (10) zur Kraftmessung ausgebildet ist.

2. Bodenaufbau nach Anspruch 1, **dadurch gekennzeichnet, dass** die Längsstreben (2) und die Querstreben (3) über Stoßverbinder und/oder Eckverbinder aneinander festgelegt sind.

3. Bodenaufbau nach Anspruch 1 oder 2, **dadurch gekennzeichnet, dass** die Halter (4) über Klemmelemente an den Längsstreben (2) und/oder Querstreben (3) lösbar festgelegt sind.

4. Bodenaufbau nach Anspruch 3, **dadurch gekennzeichnet, dass** die Klemmelemente als Nutensteine ausgebildet sind.

5. Bodenaufbau nach einem der vorhergehenden Ansprüche, **dadurch gekennzeichnet, dass** zwischen den Haltern (4) und den Bodenplatten (5) zumindest bereichsweise mindestens ein Dämpfungselement angeordnet ist.

6. Bodenaufbau nach einem der vorhergehenden Ansprüche, **dadurch gekennzeichnet, dass** die Längsstreben (2) und/oder Querstreben (3) aus Metall, insbesondere aus Aluminium, hergestellt sind.

7. Bodenaufbau nach einem der vorhergehenden Ansprüche, **dadurch gekennzeichnet, dass** die Messplatte (10) eine Vielzahl von Drucksensoren aufweist.

8. Bodenaufbau nach einem der vorhergehenden Ansprüche, **dadurch gekennzeichnet, dass** die Längsstreben (2) und/oder Querstreben (3) hinterschnittene Nuten (21, 31) aufweisen, an denen Nutensteine klemmend festlegbar sind.

9. Bodenaufbau nach Anspruch 8, **dadurch gekennzeichnet, dass** die Längsstreben (2) und/oder Querstreben (3) an mindestens zwei Seiten, vorzugsweise an allen vier Seiten jeweils eine Nut aufweisen.

10. Bodenaufbau nach einem der vorhergehenden Ansprüche, **dadurch gekennzeichnet, dass** die Halter (4) aus Metall, insbesondere Stahl oder Aluminium, hergestellt sind.

11. Bodenaufbau nach einem der vorhergehenden Ansprüche, **dadurch gekennzeichnet, dass** die Messplatte (10) an mehreren Haltern (4) kraftschlüssig festgelegt ist, insbesondere verschraubt ist, oder lose aufgelegt ist.

12. Bodenaufbau nach einem der vorhergehenden Ansprüche, **dadurch gekennzeichnet, dass** die Bodenplatten (5) ohne Messfunktion lose auf die Halter (4) aufgesteckt sind oder kraftschlüssig fixiert sind, insbesondere verschraubt sind.

13. Bodenaufbau nach einem der vorhergehenden Ansprüche, **dadurch gekennzeichnet, dass** die Längsstreben (2) und/oder die Querstreben (3) mit dem darunter angeordneten Boden verbunden sind, vorzugsweise verschraubt sind.

14. Bodenaufbau nach einem der vorhergehenden Ansprüche, **dadurch gekennzeichnet, dass** der Bodenaufbau (1) eine oder mehrere Messplatten (10) zur Kraftmessung aufweist.

15. Bodenaufbau nach einem der vorhergehenden Ansprüche, **dadurch gekennzeichnet, dass** der Bodenaufbau (1) selbsttragend stabil ausgeführt ist und mobil einsetzbar ist.

## Claims

1. Floor structure (1), in particular of a gait laboratory, having longitudinal struts (2) and transverse struts (3) which are secured to one another via releasable clamping connectors and on which there are fixed a plurality of holders (4) on which floor panels (5, 10) are deposited,
**characterized in that**
at least one of the floor panels is designed as a measuring plate (10) for force measurement.

2. Floor structure according to Claim 1, **characterized in that** the longitudinal struts (2) and the transverse struts (3) are secured to one another via butt connectors and/or corner connectors.

3. Floor structure according to Claim 1 or 2, **characterized in that** the holders (4) are releasably secured to the longitudinal struts (2) and/or transverse struts (3) via clamping elements.

4. Floor structure according to Claim 3, **characterized in that** the clamping elements are designed as groove blocks.

5. Floor structure according to one of the preceding claims, **characterized in that** at least one damping element is arranged at least in certain regions between the holders (4) and the floor panels (5).

6. Floor structure according to one of the preceding claims, **characterized in that** the longitudinal struts (2) and/or transverse struts (3) are produced from metal, in particular from aluminium.

7. Floor structure according to one of the preceding claims, **characterized in that** the measuring plate (10) has a plurality of pressure sensors.

8. Floor structure according to one of the preceding claims, **characterized in that** the longitudinal struts (2) and/or transverse struts (3) have undercut grooves (21, 31) to which groove blocks can be secured so as to have a clamping action.

9. Floor structure according to Claim 8, **characterized in that** the longitudinal struts (2) and/or transverse struts (3) each have a groove on at least two sides, preferably on all four sides.

10. Floor structure according to one of the preceding claims, **characterized in that** the holders (4) are produced from metal, in particular steel or aluminium.

11. Floor structure according to one of the preceding claims, **characterized in that** the measuring plate (10) is secured to a plurality of holders (4) in a force-fitting manner, in particular by screwing, or is loosely fitted on.

12. Floor structure according to one of the preceding claims, **characterized in that** the floor panels (5) without measuring function are plugged loosely onto the holders (4) or are fixed in a force-fitting manner, in particular by screwing.

13. Floor structure according to one of the preceding claims, **characterized in that** the longitudinal struts (2) and/or the transverse struts (3) are connected to the floor arranged below, preferably by screwing.

14. Floor structure according to one of the preceding claims, **characterized in that** the floor structure (1) has one or more measuring plates (10) for force measurement.

15. Floor structure according to one of the preceding claims, **characterized in that** the floor structure (1) has a self-supporting stable design and can be used in a mobile manner.

## Revendications

1. Structure de plancher (1) en particulier destinée à un laboratoire de passage comprenant des entretoises longitudinales (2) et des entretoises transversales (3) qui sont fixées les unes aux autres par l'intermédiaire d'organes de serrage amovibles et sur lesquelles est fixé un ensemble de supports (4) sur lesquels sont déposées des plaques de plancher (5, 10),
**caractérisée en ce qu'**
au moins l'une des plaques de plancher est réalisée sous la forme d'une plaque de mesure (10) permettant la mesure de forces.

2. Structure de plancher conforme à la revendication 1,
**caractérisée en ce que**
les entretoises longitudinales (2) et les entretoises transversales (3) sont fixées les unes aux autres par l'intermédiaire d'organes de liaison bout à bout et/ou d'organes de liaison d'angle.

3. Structure de plancher conforme à la revendication 1 ou 2,
**caractérisée en ce que**
les supports (4) sont fixés de façon amovible sur les entretoises longitudinales (2) et/ou les entretoises transversales (3) par l'intermédiaire d'éléments de serrage.

4. Structure de plancher conforme à la revendication 3,
**caractérisée en ce que**
les éléments de serrage sont réalisés sous la forme de coulisseaux.

5. Structure de plancher conforme à l'une des revendications précédentes,
**caractérisée en ce qu'**
entre les supports (4) et les plaques de plancher (5) est positionné au moins par zones au moins un élément d'amortissement.

6. Structure de plancher conforme à l'une des revendications précédentes,
**caractérisée en ce que**
les entretoises longitudinales (2) et/ou les entretoises transversales (3) sont réalisées en métal, en particulier en aluminium.

7. Structure de plancher conforme à l'une des revendications précédentes,
**caractérisée en ce que**
la plaque de mesure (10) comporte un ensemble de capteurs de pression.

8. Structure de plancher conforme à l'une des revendications précédentes,
**caractérisée en ce que**
les entretoises longitudinales (2) et/ou les entretoises transversales (3) comportent des rainures à contre-dépouille (21, 31) sur lesquelles des coulisseaux peuvent être fixés par serrage.

9. Structure de plancher conforme à la revendication 8,
**caractérisée en ce que**
les entretoises longitudinales (2) et/ou les entretoises transversales (3) comportent respectivement une rainure sur au moins deux côtés et de préférence sur leurs quatre côtés.

10. Structure de plancher conforme à l'une des revendications précédentes,
**caractérisée en ce que**
les supports (4) sont réalisés en métal, en particulier en acier ou en aluminium.

11. Structure de plancher conforme à l'une des revendications précédentes,
**caractérisée en ce que**
la plaque de mesure (10) est fixée par une liaison par la force, en particulier vissée, ou positionnée librement sur plusieurs supports (4).

12. Structure de plancher conforme à l'une des revendications précédentes,
**caractérisée en ce que**
les plaques de plancher (5) n'ayant pas de fonction de mesure sont déposées librement sur les supports (4) ou fixées par une liaison par la force, en particulier vissées.

13. Structure de plancher conforme à l'une des revendications précédentes,
**caractérisée en ce que**
les entretoises longitudinales (2) et/ou les entretoises transversales (3) sont reliées avec et de préférence vissés sur le sol situé au-dessous.

14. Structure de plancher conforme à l'une des revendications précédentes,
**caractérisée en ce qu'**
elle comporte au moins une plaque de mesure (10) pour permettre la mesure de forces.

15. Structure de plancher conforme à l'une des revendications précédentes,
**caractérisée en ce qu'**
elle est stable et autoportante et positionnable de façon mobile.
